# EUROPEAN PATENT SPECIFICATION

(11) **EP 4 526 403 B1**
(45) Date of publication and mention of the grant of the patent: **01.10.2025**
(21) Application number: 23727010.3
(22) Date of filing: 17.05.2023
(51) Int. Cl.: C10G 2/00, C10G 45/68, C10K 3/02

(54) **METHOD FOR PRODUCING SUSTAINABLE FUEL VIA CARBON MONOXIDE**
VERFAHREN ZUR HERSTELLUNG VON NACHHALTIGEM KRAFTSTOFF MITTELS KOHLENMONOXID
PROCÉDÉ DE PRODUCTION DE CARBURANT DURABLE PAR L'INTERMÉDIAIRE DE MONOXYDE DE CARBONE

(30) Priority: 19.05.2022 EP 22174406
(43) Date of publication of application: 26.03.2025
(73) Proprietor: TotalEnergies OneTech, 92400 Courbevoie (FR)
(72) Inventor: STEWART, Joseph, 7181 Seneffe (BE); VERMEIREN, Walter, 3530 Houthalen (BE)
(74) Representative: Synergy IP Group AG
(86) International application number: PCT/EP2023/063326
(87) International publication number: WO 2023/222798

(56) References cited:
- EP-A1- 3 243 891
- EP-A1- 3 992 265
- WO-A1-2013/150319
- WO-A1-2017/074843
- WO-A1-2017/108295
- WO-A1-2022/079407
- WO-A2-2004/081147
- WO-A2-2017/085603
- US-A1- 2011 105 630
- NEZAM I ET AL: "Direct aromatization of CO2via combined CO2hydrogenation and zeolite-based acid catalysis", JOURNAL OF CO2 UTILIZATION, vol. 45, 20 January 2021 (2021-01-20), pages 1 - 21, XP002807845

## Description

### Field of the invention

The invention relates to methods for producing sustainable fuel.

### Background of the invention

Climate change and the on-going energy transition makes it mandatory to replace fossil-based energy sources. In this context various aspects will be important for society to reach Net Zero by 2050 as currently desired. For example, the valorisation of alternative feedstocks is expected to contribute towards the circular economy and/or reduce the carbon dioxide (CO₂) footprint associated with the final product. However, while many alternative fuels and chemicals are sort after, drop in solutions from alternative feedstocks would allow for existing infrastructure to be maintained.

In this context even the valorisation of CO₂ as a feedstock is considered, sourced from flue gas, bio sources and even direct air capture. Upgrading of CO₂ to chemicals such as methanol has already received some attention to yield sustainable fuel for various applications. However, some applications regularly lead to enhanced requirements for the sustainable fuel. For example, aviation fuel, i.e., fuel to power aircraft, regularly requires to contain larger hydrocarbons and aromatics. This is because larger hydrocarbons and aromatics regularly improve the cold flow properties of the aviation fuel. Such improved cold flow properties prevent fuel freezing at low temperatures of for example -40°C, which are typical for cruising altitudes of the powered aircrafts. In attempts to upgrade CO₂, larger hydrocarbons and aromatics are however regularly more difficult to achieve.

In a different approach, bio conversion and syngas upgrading via Fischer-Tropsch (FT) are also considered as an alternative to a fossil fuel feedstock. Such routes may appear attractive for diesel and gasoline production. However, they are not suitable for 100% aviation fuel, as they crucially lack aromatic content and hence are applied as a blend.

Thus far, there are no established routes of producing a 100% sustainable aviation fuel by bio conversion or syngas upgrading via FT.

Further in the search for sustainable fuel and in particular in the search for aviation fuel, hydrodeoxygenation of fatty acids/triglycerides, CO₂- or bio-sourced syngas upgrading, or bio-olefin oligomerisation have been researched at various Technology Readiness Levels (TRLs). However, for each of these routes, the major product is regularly long hydrocarbon chains and is typically only suitable for up to 50% blend to meet requirements for aviation fuel. This is because these routes do regularly not yield aromatics which are however also required to improve the cold flow properties of aviation fuel.

EP 3 243 891 discloses a method for producing a fuel comprising the steps of converting CO₂ into hydrocarbons via a reverse water gas shift reaction and Fischer-Tropsch reaction. Nezam I et al., Journal of CO2 Utilization, vol. 45, 20 January 2022, pages 1-21, discloses a method for producing a fuel comprising the steps of converting CO₂ into C₁-C₆ olefins using a bifunctional catalyst and converting C₁-C₆ hydrocarbons into aromatics. WO 2017/074843 discloses a method comprising the steps of converting CO₂ into CO using a reverse water gas shift catalyst at 600°C, optionally separating at least of portion of H₂O from the product stream by condensation to provide as purified syngas and converting CO into light olefins.

Overall, there remains a general desire for an improved method for producing sustainable fuel.

### Problem underlying the invention

It is an object of the present invention to provide a method for producing sustainable fuel which at least partially overcomes the drawbacks encountered in the art.

It is in particular an object of the present invention to provide a method for producing sustainable fuel which reduces the CO₂ footprint associated with the produced sustainable fuel.

It is furthermore an object of the present invention to provide a method for producing sustainable fuel which allows for existing infrastructure to be maintained.

It is moreover an object of the present invention to provide a method for producing sustainable fuel which has an improved energy efficiency and/or an improved cost efficiency.

It is also an object of the present invention to provide a method for producing sustainable fuel which has an improved carbon efficiency, i.e., a method which minimizes carbon dioxide equivalents emissions to its output.

It is additionally an object of the present invention to provide a method for producing sustainable fuel which has an increased aromatics content.

It is in particular an object of the present invention to provide a method for producing sustainable fuel which meets the requirements for aviation fuel.

### Disclosure of the invention

Surprisingly, it has been found that the problem underlying the invention is overcome by methods according to the claims. Further embodiments of the invention are outlined throughout the description.

Subject of the invention is a method for producing sustainable fuel according to claim 1, comprising the steps:
(i) converting CO₂ into CO using a reverse water gas shift catalyst,
(ii) converting CO from step (i) into C₁-C₆ hydrocarbons using a Fischer-Tropsch catalyst, and
(iii) converting C₁-C₆ hydrocarbons from step (ii) into aromatics using a zeolite-based catalyst.
further comprising a cooling step in which CO from step (i) is cooled before being converted in step (ii).

Logically, steps (i), (ii) and (iii) are carried out in the given order, i.e., first step (i), thereafter step (ii) and thereafter step (iii). However, additional steps before or after each of steps (i), (ii) and (iii) may also be comprised by the method according to the present invention.

In step (i), carbon dioxide (CO₂) is converted into carbon monoxide (CO). The conversion occurs in the presence of a reverse water gas shift catalyst. As used herein, a reverse water gas shift catalyst promotes a reverse water gas shift reaction. In other words, the reverse water gas shift catalyst lowers the activation energy of a reverse water gas shift reaction. As used herein, a reverse water gas shift reaction is a reaction which comprises a reaction of CO₂ with hydrogen (H₂) to yield CO and water (H₂O). The reverse water gas shift reaction, and hence step (i), comprises the following reaction: CO₂ + H₂ → CO + H₂O

In step (ii), CO is converted into short hydrocarbons, namely C₁-C₆ hydrocarbons. C₁-C₆ hydrocarbons are compounds containing at least one and at most six carbon atoms, i.e., 1, 2, 3, 4, 5 or 6 carbon atoms, and additionally hydrogen. The C₁-C₆ hydrocarbons can be saturated and/or unsaturated hydrocarbons. Saturated C₁-C₆ hydrocarbons are regularly linear, branched or cyclic alkyls. Unsaturated C₁-C₆ hydrocarbons, more precisely unsaturated C₂-C₆ hydrocarbons, are regularly linear, branched or cyclic alkenyls or alkynyls. The C₁-C₆ hydrocarbons may comprise heteroatoms like oxygen (O), sulfur (S), nitrogen (N) or phosphorous (P). However, according to the present invention, the C₁-C₆ hydrocarbons are preferably free from heteroatoms like oxygen (O), sulfur (S), nitrogen (N) and phosphorous (P).

In step (ii), CO is converted using a Fischer-Tropsch catalyst (or FT-catalyst), that is, in the presence of a Fischer-Tropsch catalyst. As used herein, a Fischer-Tropsch catalyst promotes a Fischer-Tropsch reaction. In other words, the Fischer-Tropsch catalyst lowers the activation energy of a Fischer-Tropsch reaction. As used herein, a Fischer-Tropsch reaction is a reaction which comprises a reaction of CO with hydrogen (H₂) to yield at least the C₁-C₆ hydrocarbons. During the reaction, the hydrocarbons regularly grow in a repeated sequence in which hydrogen atoms are added to carbon and oxygen, the C-O bond of CO is split and a new C-C bond is formed. For one -CH₂- group, the reaction can be given as follows:

CO + 2 H₂ → (CH₂) + H₂O

Such a Fischer-Tropsch reaction can for example comprise the following reaction steps:
associative adsorption of CO by the FT-catalyst;
splitting of the C-O bond;
dissociative adsorption of 2 H₂ by the FT-catalyst;
transfer of 2 H to the oxygen to yield H₂O;
desorption of H₂O from the FT-catalyst;
transfer of 2 H to the carbon to yield CH₂.

In step (iii), C₁-C₆ hydrocarbons from step (ii) are converted into aromatics. As used herein, aromatics are aromatic in the sense of the IUPAC Gold Book. More specifically, aromatics are cyclically conjugated molecular entities with a stability (due to delocalization) significantly greater than that of a hypothetical localized structure (e.g., Kekulé structure). Such cyclically conjugated molecular entities have aromaticity. A method for determining aromaticity can in particular be the observation of diatropicity in the ¹H-NMR spectrum.

In step (iii), C₁-C₆ hydrocarbons from step (ii) are converted using a zeolite-based catalyst, that is, in the presence of a zeolite-based catalyst. As used herein, zeolite-based means that the catalyst comprises zeolite. The zeolite-based catalyst is preferably composed of ≥ 50 wt.%, more preferably of ≥ 60 wt.%, still more preferably of ≥ 70 wt.%, even more preferably of ≥ 80 wt.% and in particular preferably of ≥ 90 wt.% of zeolite; the weight percentages are based on the total weight of the zeolite-based catalyst. In a particular case, the zeolite-based catalyst consists of zeolite. As used herein, zeolite is given the same meaning as usual in the art. In particular, a zeolite has an aluminosilicate matrix with a tetrahedral arrangement of silicon (Si⁴⁺) and aluminium (Al³⁺) cations surrounded by four oxygen anions (O²⁻). This regularly results in a macromolecular three-dimensional structure of SiO₂ and AlO₂ tetrahedral building blocks. As the AlO₂ tetrahedral building blocks are negatively charged, zeolites regularly comprise additional charge-compensating cations, e.g., alkali metal cations, alkaline earth metal cations, protons and/or ammonia cations.

In steps (i) to (iii), the afore-listed catalysts are used, namely, a reverse water gas shift catalyst, a Fischer-Tropsch catalyst and a zeolite-based catalyst. According to the present invention, all these catalysts are used in solid form. Here, solid form refers to the aggregation state of the respective catalysts, in particular under normal conditions of 298.15 K and 101.3 kPa.

The method for producing sustainable fuel according to the present invention uses CO₂ as a feedstock, which is at least partially converted and is hence not emitted to the environment. The inventive method can thus help to reduce the CO₂ footprint associated with the produced sustainable fuel. Herein, the CO₂ footprint refers to the amount of carbon dioxide released into the atmosphere.

The method for producing sustainable fuel according to the present invention can be carried out in one or more reaction vessels, especially reactors, which have previously been used for conversion of fossil feedstock. The inventive method may thus allow for existing infrastructure to be maintained.

The method for producing sustainable fuel according to the present invention combines ways of producing larger hydrocarbons and aromatics and yields a sustainable fuel comprising such larger hydrocarbons and aromatics in one single continuous reaction sequence. The inventive method can thereby lead to improved energy efficiency and/or improved cost efficiency of the fuel production.

The method for producing sustainable fuel according to the present invention combines ways of producing larger hydrocarbons and aromatics and regularly yields a sustainable fuel comprising such larger hydrocarbons and aromatics. The inventive method can thereby produce sustainable fuel which meets the requirements for aviation fuel.

The method according to the present invention further comprises a cooling step in which CO from step (i) is cooled before being converted in step (ii). The cooling step lowers requirements to extract heat from the reaction vessel, like a reactor, in which step (ii) is carried out, without however disadvantageously reducing the conversion rate of CO in step (ii). Preferably, during the cooling step between step (i) and step (ii) liquid water is removed resulting in the water-poor CO stream. The Fischer-Tropsch step (ii) is a highly exothermic reaction step and tends to increase the temperature in the reactor, requiring cooling of the reactor content. Hence it is preferred that the CO stream enters step (ii) cooler than it leaves step (i). Moreover, when significant amounts of water are still present in the CO stream, the Fischer-Tropsch catalyst will perform the water-gas shift reaction (CO + H₂O → CO₂ + H₂), being also exothermic and producing heat. It is preferred that the CO stream is poor in water content. Further, the conversion rate of CO in step (ii) may even increase. The cooling step thus further improves the energy efficiency and/or the cost efficiency of the fuel production. In this context, cooling of the CO naturally means that the temperature of the CO obtained in step (i) is lowered before the CO is used in step (ii) for a conversion into C₁-C₆ hydrocarbons. Hence, also the notation T_{(CO}, _{step (i))} > T_{(CO, step (ii))} can be used to indicate that there is an active cooling of the CO between step (i) and step (ii). Moreover, with a maintained or even increased conversion rate of CO in step (ii) the conversion thereof into aromatics in subsequent step (iii) may also be improved, i.e., the aromatics yield can be increased.

In the method according to the present invention, the CO from step (i) is cooled (in the cooling step) from a temperature of ≥ 500°C to a temperature of ≤ 350°C before being converted in step (ii), preferably from a temperature of ≥ 500°C to a temperature of ≤ 300°C and more preferably from a temperature of ≥ 500°C to a temperature of ≤ 250°C. An advantageously efficient conversion of CO₂ into CO in the reverse water gas shift reaction in step (i) will regularly lead to a product stream of increased temperature, in particular an increased temperature of ≥ 500°C. It has been found that in order to increase the efficiency of the Fischer-Tropsch reaction and to increase the yield of the C₁ to C₆ hydrocarbons in step (ii), in particular the yield of unsaturated C₂ to C₆ hydrocarbons, it is advantageous to lower the temperature of CO from 500°C or more to 350°C or less, even better to 300°C or less and especially to 250°C or less. A respective lowering of the temperature of the CO can then ultimately also increase the yield of aromatics obtained in step (iii).

In the context of the cooling step, it is especially preferred that step (i) is carried out in a first reactor and that step (ii) is carried out in a physically separated second reactor, wherein the cooling takes place between the CO leaving the first reactor and the CO entering the second reactor. It is especially preferred that in the cooling step liquid water is removed from the CO stream.

It is preferred that in a method according to the present invention, in step (i) the CO₂ is at least partially reacted with H₂, and/or in step (ii) the CO is at least partially reacted with H₂. A reaction with H₂ in either step can lead to an increased conversion of CO₂ and/or CO, which can further reduce the CO₂ footprint. Additionally, an increased conversion of CO₂ and/or CO can further improve the energy efficiency and/or the cost efficiency of the fuel production.

It is preferred that in a method according to the present invention, the reverse water gas shift catalyst comprises Fe, Co, Cu, Cr, Ni, Ir, Mn or mixtures thereof, preferably in oxidic form (Fe-oxide, Co-oxide, Cu-oxide, Cr-oxide, Ni-oxide, Ir-oxide, Mn-oxide, or mixtures thereof) or in metallic form supported on metal oxide(s) or supported on carbon. It is preferred that the reverse water gas shift catalyst comprises Ni, even more preferably comprises Ni supported on metal oxide, in particular Ni/Al₂O₃. It is also preferred that the reverse water gas shift catalyst comprises Fe, even more preferably comprises an Fe-oxide, in particular Fe₂O₃ or Fe₃O₄. The use of a reverse water gas shift catalyst which comprises Fe, Co, Cu, Cr, Ni, Ir, Mn or mixtures thereof can lead to an increased conversion of CO₂ in step (i), which can further reduce the CO₂ footprint. Additionally, such a reverse water gas shift catalyst comprising Fe, Co, Cu, Cr, Ni, Ir, Mn or mixtures thereof may already be used in existing infrastructure. Hence, such a reverse water gas shift catalyst comprising Fe, Co, Cu, Cr, Ni, Ir, Mn or mixtures thereof can help to maintain existing infrastructure. The mentioned effects can be particularly pronounced when the reverse water gas shift catalyst comprises either Ni supported on metal oxide, in particular Ni/Al₂O₃, or an Fe-oxide, in particular Fe₂O₃ or Fe₃O₄.

It is similarly preferred that in a method according to the present invention, the reverse water gas shift catalyst comprises Fe, Co, Cu, Cr, Ni, Ir, Mn or mixtures thereof in the form of sulphide(s) or carbide(s).

It is further preferred that in a method according to the present invention, the reverse water gas shift catalyst comprises Fe, Co, Cu, Cr, Ni, Ir, Mn or mixtures thereof, preferably in the form of oxides, sulphides or carbides, or preferably in metallic form supported on metal oxide(s) or supported on carbon, wherein the water gas shift catalyst further comprises a promotor metal species selected from alkali metals and alkaline earth metals.

It is also preferred that in a method according to the present invention, the reverse water gas shift catalyst is selected from
- oxide catalysts, preferably selected from CeO₂, CuO, ZnO, Al₂O₃, Fe₂O₃, Cr₂O₃, In₂O₃, and MnO₂,
- composite oxides, preferably selected from CuO/ZnO/Al₂O₃, NiO/CeO₂, ZnO/Al₂O₃, ZnO/Cr₂O₃, CuOₓ/CeO₂, and In₂O₃-CeO₂,
- spinel oxides, preferably selected from ZnAl₂O₄, ZnCr₂O₄, Cu Al₂O₄, and Co Al₂O₄,
- solid solution oxides, preferably selected from ZnₓZr₁₋ₓO_{2-y}, Ce_{0.5}Zr_{0.5}O₂, NixCe_{0.75}Zr_{0.25-x}O₂, and
- perovskite-type oxides, preferably selected from BaZr_{0.8}Y_{0.16}Zn_{0.04}O3, La_{0.75}Sr_{0.25}CoO_{3-d}, La_{0.75}Sr_{0.25}FeO₃, La_{0.75}Sr_{0.25}Fe_{1-Y}CuYO₃, LaNiO₃, La_{0.9}Sr_{0.1}NiO_{3+d}, La_{0.9}Sr_{0.1}FeO_{3-d}, La_{0.9}Sr_{0.1}Ni_{0.5}Fe_{0.5}O_{3-d}, La_{0.75}Sr_{0.25}Cr_{0.5}Mn_{0.5}O_{3-d}, and SrCe_{0.9}Y_{0.1}O_{3-d}.

It is also preferred that in a method according to the present invention, the reverse water gas shift catalyst is selected from metals, which metals are preferably selected from Pt, Pd, Au, Rh, Ru, Cu, Ni, Re, Co, Fe, and Mo, which are immobilized on a metal oxide support material, which support material is preferably selected from CeO₂, TiO₂, Al₂O₃, ZnO, ZrO₂, and SiO₂. It is additionally preferred that such a water gas shift catalyst further comprises a promotor metal species selected from alkali metals and alkaline earth metals.

It is preferred that in a method according to the present invention, the Fischer-Tropsch catalyst comprises Fe and/or Co, preferably Co. It is more preferred that the Fischer-Tropsch catalyst comprises Fe or Co which is supported on an oxide, in particular Co supported on an oxide. The use of a Fischer-Tropsch catalyst which comprises Fe or Co can lead to an increased conversion of CO in step (ii), which can further improve the energy efficiency and/or the cost efficiency of the fuel production. Additionally, such a Fischer-Tropsch catalyst comprising Fe or Co may already be used in existing infrastructure. Hence, such a Fischer-Tropsch catalyst comprising Fe or Co can help to maintain existing infrastructure. The mentioned effects can be particularly pronounced when the Fischer-Tropsch catalyst comprises Co. Optionally, the Fe comprised by the Fischer-Tropsch catalyst is metallic Fe, the Co comprised by the Fischer-Tropsch catalyst is metallic Co, or the Fe comprised by the Fischer-Tropsch catalyst is metallic Fe and the Co comprised by the Fischer-Tropsch catalyst is metallic Co.

It is preferred that in a method according to the present invention, the zeolite-based catalyst in step (iii) comprises MFI-type zeolite (especially a ZSM-5 zeolite or an HZSM-5 zeolite), a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof. More preferably, the zeolite-based catalyst in step (iii) comprises a ZSM-5 zeolite or an HZSM-5 zeolite, particularly preferable an HZSM-5 zeolite (an HZSM-5 zeolite is a proton-exchanged form of a ZSM-5 zeolite). The listed zeolite types are indicated here by the codes attributed by the International Zeolite Association. The use of a zeolite-based catalyst which comprises a zeolite of the listed types can lead to an increased conversion of C₁-C₆ hydrocarbons into aromatics in step (iii), which can further improve the energy efficiency and/or the cost efficiency of the fuel production. Additionally, such a zeolite-based catalyst of the listed types may already be used in existing infrastructure. Hence, such a zeolite-based catalyst of the listed types can help to maintain existing infrastructure. The mentioned effects can be particularly pronounced when the zeolite-based catalyst in step (iii) comprises a ZSM-5 zeolite or an HZSM-5 zeolite, in particular an HZSM zeolite. Herein, a ZSM-5 zeolite and an HZSM-5 zeolite may be combinedly referred to as (H)ZSM-5 zeolite.

It is preferred that in a method according to the present invention, the zeolite-based catalyst in step (iii) comprises a metal-modified zeolite. As used herein, "metal-modified" means that the zeolite contains metal cations different from alkali metal cations and alkaline earth metal cations. Preferred metal cations are Zn-cations, Ga-cations, Ag-cations, Mo-cations and/or Re-cations. Accordingly, it is particularly preferred that the zeolite-based catalyst in step (iii) comprises a Zn-modified zeolite, a Ga-modified zeolite, an Ag-modified zeolite, an Mo-modified zeolite and/or a Re-modified zeolite. The metal-modified zeolite can be an MFI-type zeolite, a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof. The use of a metal-modified zeolite can lead to an increased conversion of C₁-C₆ hydrocarbons into aromatics in step (iii), which can further improve the energy efficiency and/or the cost efficiency of the fuel production. Additionally, such a metal-modified zeolite may already be used in existing infrastructure. Hence, such a metal-modified zeolite can help to maintain existing infrastructure.

It is preferred that in a method according to the present invention, another zeolite-based catalyst is present in step (i), more preferably a metal-modified zeolite, still more preferably a zeolite selected from a Zn-modified zeolite, a Ga-modified zeolite, an Ag-modified zeolite, an Mo-modified zeolite and/or a Re-modified zeolite. The "another" zeolite-based catalyst is different from the zeolite-based catalyst used in step (iii), i.e., it is a further zeolite-based catalyst. The presence of such a further zeolite-based catalyst in step (i) may further promote the reverse water gas shift reaction, which is especially the case for a metal-modified zeolite.

It is preferred that in a method according to the present invention, step (ii) additionally produces saturated C7+ hydrocarbons, preferably saturated C8+ hydrocarbons. Saturated C7+ hydrocarbons are saturated hydrocarbons which contain seven or more (≥7) carbon atoms. Saturated C8+ hydrocarbons are saturated hydrocarbons which contain eight or more (≥8) carbon atoms. Saturated C7+ hydrocarbons are particularly suitable for combustion in aircraft engines. Accordingly, when the saturated hydrocarbons comprise saturated C7+ hydrocarbons, the inventive method can be particularly suitable for producing sustainable fuel which meets the requirements for aviation fuel. The mentioned effect can be particularly pronounced when the saturated hydrocarbons comprise saturated C8+ hydrocarbons.

In the method according to the present invention, the C₁-C₆ hydrocarbons comprise ethylene, propylene and/or butylene. When the C₁-C₆ hydrocarbons produced in step (ii) comprise ethylene, propylene and/or butylene, the subsequent conversion thereof into aromatics in step (iii) can have a higher conversion rate and/or may require less energy. Accordingly, when the C₁-C₆ hydrocarbons comprise ethylene, propylene and/or butylene, the energy efficiency and/or the cost efficiency of the fuel production can be improved.

It is preferred that in a method according to the present invention, methane (CH₄) is produced in step (i) which is subsequently at least partially converted into aromatics in step (iii). It is known that a conversion of CO₂ into CO over a reverse water gas shift catalyst may yield CH₄ as a by-product. Like the C₁-C₆ hydrocarbons produced in step (ii), which may also comprise CH₄, additional CH₄ produced in step (i) can subsequently be at least partially converted into aromatics in step (iii). This additional synthesis of aromatics in the inventive method can improve the aromatics content of the produced sustainable fuel, which can further help to meet the requirements for aviation fuel. Moreover, the by-product CH₄ from step (i) is not lost, but is rather valorised.

It is preferred that in a method according to the present invention, step (i) is performed at a temperature of 250 to 1000°C, more preferably of 300 to 750°C and still more preferably of 300 to 600°C. An increased temperature of 250 to 1000°C in step (i) can lead to an increased conversion of the CO₂. This can help to further reduce the CO₂ footprint of the sustainable fuel produced by the inventive method. At the same time, too high temperatures may lead to a reduced energy efficiency and/or cost efficiency of the fuel production. Temperatures of 300 to 750°C and in particular of 300 to 600°C are therefore particularly preferred.

It is preferred that in a method according to the present invention, step (i) is performed at an absolute pressure of 0.1 to 10 MPa, more preferably of 1 to 4 MPa. An absolute pressure of 0.1 to 10 MPa in step (i) can lead to an increased conversion of the CO₂. This can help to further reduce the CO₂ footprint of the sustainable fuel produced by the inventive method. At the same time, too high pressures may lead to a reduced energy efficiency and/or cost efficiency of the fuel production. Absolute pressures of 1 to 4 MPa are therefore particularly preferred.

It is preferred that in a method according to the present invention, a feed is fed in step (i) to the reverse water gas shift catalyst which has a molar ratio of hydrogen to carbon oxides, i.e., a molar ratio H₂:COₓ, of 0.5 to 12, more preferably of 1 to 3, with x being 1 and/or 2, with x preferably being 2. With such a molar ratio of hydrogen to carbon oxides of 0.5 to 12 an increased conversion of CO₂ in step (i) may be achieved which can help to further reduce the CO₂ footprint of the sustainable fuel produced by the inventive method. These effects can be particularly pronounced when the ratio of hydrogen to carbon oxides is 1 to 3.

It is preferred that in a method according to the present invention, H₂O is produced in step (i) to yield a mixture comprising CO and H₂O, wherein the H₂O is at least partially separated from the CO in the cooling step. The separation of H₂O can help to maintain or even increase the conversion rate of CO in step (ii). The separation of H₂O can thus further improve the energy efficiency and/or the cost efficiency of the fuel production.

It is preferred that in a method according to the present invention, in step (i) a feed comprising the CO₂ is fed to the reverse water gas shift catalyst, wherein the feed is substantially free of CO. When the feed is substantially free of CO, preferably free of CO, the absent CO cannot interfere with the reverse water gas shift reaction. Accordingly, a substantial absence of CO and in particular a complete absence of CO in the feed fed to the reverse water gas shift catalyst can lead to an increased conversion of CO₂ in step (i), which can further reduce the CO₂ footprint.

In a method according to the present invention, step (ii) is performed at a temperature of 200 to 350°C, preferably of 250 to 350°C. An increased temperature in step (ii) can lead to an increased conversion of the CO. It is preferred that in a method according to the present invention, step (iii) is performed at a temperature of 200 to 500°C, more preferably of 250 to 350°C. An increased temperature of 200 to 500°C in step (iii) can lead to an increased conversion of the C₁-C₆ hydrocarbons. At the same time, too high temperatures may lead to a reduced energy efficiency and/or cost efficiency of the fuel production. A temperature of 250 to 350°C is therefore respectively preferred.

It is preferred that in a method according to the present invention, step (ii) and/or (iii) is performed at an absolute pressure of 0.1 to 5 MPa, more preferably of 1 to 3 MPa. An absolute pressure of 0.1 to 5 MPa in step (ii) can lead to an increased conversion of the CO. An absolute pressure of 0.1 to 5 MPa in step (iii) can lead to an increased conversion of the unsaturated hydrocarbons. At the same time, too high pressures may lead to a reduced energy efficiency and/or cost efficiency of the fuel production. Absolute pressures of 1 to 3 MPa are therefore particularly preferred.

It is preferred that in a method according to the present invention,
in step (i) the CO₂ is reacted with H₂ at a temperature of 250 to 1000°C using a reverse water gas shift catalyst which comprises Ni/Al₂O₃ to yield a mixture comprising CO and H₂O,
the H₂O is at least partially separated from the CO in a subsequent cooling step before the CO is converted in step (ii),
in step (ii) CO from step (i) is reacted with H₂ using a Fischer-Tropsch catalyst which comprises Co to yield saturated C8+ hydrocarbons and unsaturated hydrocarbons which comprise at least one of ethylene, propylene and/or butylene, and
in step (iii) the zeolite-based catalyst comprises an MFI-type or a BEA-type zeolite.

Such a preferred method for producing sustainable fuel according to the present invention can in particular help to reduce the CO₂ footprint associated with the produced sustainable fuel, may allow for existing infrastructure to be maintained, can lead to an improved energy efficiency and/or an improved cost efficiency of the fuel production and can produce sustainable fuel which meets the requirements for aviation fuel.

### Brief description of the drawings

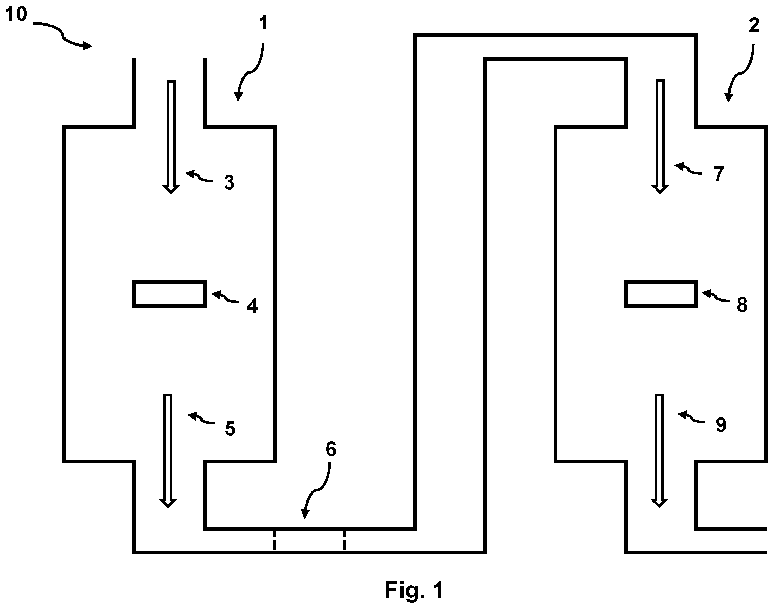

Fig. 1 shows an exemplary reactor system in which a method according to the present invention can be carried out.

### Exemplary embodiment

The present invention is further described with reference to the accompanying Fig. 1 which shows an exemplary reactor system 10. The reactor system 10 has two reactors, namely a first reactor 1 and a second reactor 2, which are in fluid communication with each other. A first feed 3 is fed to the first reactor 1. Feed 3 comprises CO₂ and H₂, and depending on the source of the CO₂ potentially minor amounts of CO (typically however 0% CO) and light hydrocarbons. The first reactor 1 contains a first catalyst bed 4 which contains Ni/Al₂O₃ as a reverse water gas shift catalyst. The first reactor bed 1 is operated at elevated temperatures between 250 and 1000°C and absolute pressures between 0.1 and 10 MPa. The thereby produced CO and H₂O are contained in a first product 5, together with unreacted H₂ and potentially unreacted CO₂. Methane (CH₄) in low amounts may also be produced in reactor 1, in which case the first product 5 additionally contains CH₄. The first product 5 is withdrawn from reactor 1 and is sent to reactor 2. In-between reactor 1 and reactor 2, a cooling device 6 may be arranged. The cooling device 6 cools the first product 5 to the reaction temperature of the second reactor 2. The cooling device 6 further separates at least some of the H₂O produced in the first reactor 1 to give the second feed 7 which is fed to the second reactor 2. The second feed 7 contains CO, H₂ and potentially CO₂ and/or CH₄. The second reactor 2 contains a second catalyst bed 8 which contains a Co-based Fischer-Tropsch catalyst (metallic Co on support) and an HZSM-5 zeolite catalyst. The second reactor 2 is operated at elevated temperatures between 200 and 500°C and absolute pressures between 0.1 and 5 MPa. In the second reactor 2, unreacted H₂ and CO generated from the first reactor are converted by the Fischer-Tropsch catalyst to C₁-C₆ hydrocarbons, which contain C₂-C₄ alkenyls, and additionally to saturated C7+ hydrocarbons. The C₁-C₆ hydrocarbons are converted to aromatics by the HZSM-5 zeolite catalyst. As a result, a second product 9 is obtained which contains long hydrocarbons and aromatics, and potentially residual CO, H₂, CO₂ and/or CH₄. The second product 9 can be withdrawn from the second reactor as the overall product, i.e., as a sustainable (raw) fuel for further use or refinement thereof.

### List of reference signs

- 1:: First reactor
- 2:: Second reactor
- 3:: First feed
- 4:: First catalyst bed
- 5:: First product
- 6:: Cooling device
- 7:: Second feed
- 8:: Second catalyst bed
- 9:: Second product
- 10:: Reactor system

## Claims

1. A method for producing sustainable fuel, comprising the steps:
(i) converting CO₂ into CO using a reverse water gas shift catalyst,
(ii) converting CO from step (i) into C₁-C₆ hydrocarbons using a Fischer-Tropsch catalyst, and
(iii) converting C₁-C₆ hydrocarbons from step (ii) into aromatics using a zeolite-based catalyst,
wherein the C₁-C₆ hydrocarbons comprise ethylene, propylene and/or butylene,
further comprising a cooling step in which CO from step (i) is cooled before being converted in step (ii), wherein the CO from step (i) is cooled from a temperature of ≥ 500°C to a temperature of ≤ 350°C before being converted in step (ii),
wherein step (ii) is performed at a temperature of 200 to 350°C.

2. The method according to claim 1, wherein step (i) is performed at a temperature of 250 to 1000°C.

3. The method according to claim 1 or 2, wherein H₂O is produced in step (i) to yield a mixture comprising CO and H₂O, wherein the H₂O is at least partially separated from the CO in the cooling step.

4. The method according to any of the preceding claims, wherein the reverse water gas shift catalyst comprises Fe, Co, Cu, Cr, Ni, Ir, Mn or mixtures thereof.

5. The method according to any of the preceding claims, wherein the Fischer-Tropsch catalyst comprises Fe and/or Co, preferably Co.

6. The method according to any of the preceding claims, wherein the zeolite-based catalyst in step (iii) comprises an MFI-type zeolite, a CHA-type zeolite, a BEA-type zeolite, a MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof.

7. The method according to any of the preceding claims, wherein another metal-modified zeolite-based catalyst is present in step (i).

8. The method according to any of the preceding claims, wherein step (ii) additionally produces saturated C7+ hydrocarbons, preferably saturated C8+ hydrocarbons.

9. The method according to any of the preceding claims, wherein in step (i) a feed comprising the CO₂ is fed to the reverse water gas shift catalyst, wherein the feed is substantially free of CO.

10. The method according to any of the preceding claims,
wherein in step (i) the CO₂ is reacted with H₂ at a temperature of 250 to 1000°C using a reverse water gas shift catalyst which comprises Ni/Al₂O₃ to yield a mixture comprising CO and H₂O,
wherein the H₂O is at least partially separated from the CO in a subsequent cooling step before the CO is converted in step (ii),
wherein in step (ii) CO from step (i) is reacted with H₂ using a Fischer-Tropsch catalyst which comprises Co to yield saturated C8+ hydrocarbons and unsaturated hydrocarbons which comprise at least one of ethylene, propylene and/or butylene, and
wherein in step (iii) the zeolite-based catalyst comprises an MFI-type zeolite or a BEA-type zeolite.

## Patentansprüche

1. Verfahren zur Herstellung von nachhaltigem Kraftstoff, umfassend die Schritte:
(i) Umwandlung von CO₂ in CO unter Verwendung eines Katalysators für reverse Wassergas-Shift-Reaktion,
(ii) Umwandlung von CO aus Schritt (i) in C₁-C₆-Kohlenwasserstoffe unter Verwendung eines Fischer-Tropsch-Katalysators und
(iii) Umwandlung von C₁-C₆-Kohlenwasserstoffen aus Schritt (ii) in Aromaten unter Verwendung eines Katalysators auf Zeolithbasis,
wobei die C₁-C₆-Kohlenwasserstoffe Ethylen, Propylen und/oder Buten umfassen,
weiterhin umfassend einen Kühlschritt, in dem CO aus Schritt (i) vor der Umwandlung in Schritt (ii) gekühlt wird, wobei das CO aus Schritt (i) vor der Umwandlung in Schritt (ii) von einer Temperatur von ≥ 500°C auf eine Temperatur von ≤ 350°C gekühlt wird,
wobei Schritt (ii) bei einer Temperatur von 200 bis 350°C durchgeführt wird.

2. Verfahren nach Anspruch 1, wobei Schritt (i) bei einer Temperatur von 250 bis 1000°C durchgeführt wird.

3. Verfahren nach Anspruch 1 oder 2, wobei in Schritt (i) H₂O erzeugt wird, um ein Gemisch aus CO und H₂O zu erhalten, wobei das H₂O in dem Kühlschritt zumindest teilweise von dem CO getrennt wird.

4. Verfahren nach einem der vorhergehenden Ansprüche, wobei der Katalysator für reverse Wassergas-Shift-Reaktion Fe, Co, Cu, Cr, Ni, Ir, Mn oder Mischungen davon umfasst.

5. Verfahren nach einem der vorhergehenden Ansprüche, wobei der Fischer-Tropsch-Katalysator Fe und/oder Co, vorzugsweise Co, umfasst.

6. Verfahren nach einem der vorhergehenden Ansprüche, wobei der Katalysator auf Zeolithbasis in Schritt (iii) einen Zeolith vom MFI-Typ, einen Zeolith vom CHA-Typ, einen Zeolith vom BEA-Typ, einen Zeolith vom MOR-Typ, einen Zeolith vom FAU-Typ, einen Zeolith vom MEL-Typ, einen Zeolith vom FER-Typ, einen Zeolith vom MTT-Typ, einen Zeolith vom TON-Typ, einen Zeolith vom ERI-Typ, einen Zeolith vom MTW-Typ, einen Zeolith vom MWW-Typ oder eine Mischung davon umfasst.

7. Verfahren nach einem der vorhergehenden Ansprüche, wobei in Schritt (i) ein weiterer metallmodifizierter Katalysator auf Zeolithbasis vorliegt.

8. Verfahren nach einem der vorhergehenden Ansprüche, wobei Schritt (ii) zusätzlich gesättigte C7+-Kohlenwasserstoffe, vorzugsweise gesättigte C8+-Kohlenwasserstoffe, erzeugt.

9. Verfahren nach einem der vorhergehenden Ansprüche, wobei in Schritt (i) ein das CO₂ enthaltendes Einsatzmaterial dem Katalysator für reverse Wassergas-Shift-Reaktion zugeführt wird, wobei das Einsatzmaterial im Wesentlichen frei von CO ist.

10. Verfahren nach einem der vorhergehenden Ansprüche,
wobei in Schritt (i) das CO₂ mit H₂ bei einer Temperatur von 250 bis 1000°C unter Verwendung eines Katalysators für reverse Wassergas-Shift-Reaktion, der Ni/Al₂O₃ umfasst, zu einer Mischung umfassend CO und H₂O umgesetzt wird,
wobei das H₂O in einem nachfolgenden Kühlschritt zumindest teilweise vom CO getrennt wird, bevor das CO in Schritt (ii) umgewandelt wird,
wobei in Schritt (ii) das CO aus Schritt (i) mit H₂ unter Verwendung eines Fischer-Tropsch-Katalysators, der Co umfasst, umgesetzt wird, um gesättigte C8+-Kohlenwasserstoffe und ungesättigte Kohlenwasserstoffe die mindestens eines von Ethylen, Propylen und/oder Buten umfassen, zu erhalten, und
wobei in Schritt (iii) der Zeolith-basierte Katalysator einen Zeolith vom MFI-Typ oder einen Zeolith vom BEA-Typ umfasst.

## Revendications

1. Procédé de production de carburant durable, comprenant les étapes suivantes :
(i) conversion du CO₂ en CO à l'aide d'un catalyseur de réaction de gaz à l'eau inverse,
(ii) conversion du CO de l'étape (i) en hydrocarbures en C₁-C₆ à l'aide d'un catalyseur Fischer-Tropsch, et
(iii) conversion des hydrocarbures en C₁-C₆ de l'étape (ii) en aromatiques à l'aide d'un catalyseur à base de zéolite,
dans lequel les hydrocarbures en C₁-C₆ comprennent l'éthylène, le propylène et/ou le butylène,
comprenant en outre une étape de refroidissement dans laquelle le CO de l'étape (i) est refroidi avant d'être converti dans l'étape (ii), dans laquelle le CO de l'étape (i) est refroidi d'une température ≥ 500°C à une température ≤ 350°C avant d'être converti dans l'étape (ii),
dans laquelle l'étape (ii) est réalisée à une température de 200 à 350°C.

2. . Procédé selon la revendication 1, dans lequel l'étape (i) est réalisée à une température de 250 à 1000°C.

3. . Procédé selon la revendication 1 ou 2, dans laquelle H₂O est produit à l'étape (i) pour donner un mélange comprenant du CO et H₂O, H₂O étant au moins partiellement séparé du CO dans l'étape de refroidissement.

4. . Procédé selon l'une quelconque des revendications précédentes, dans laquelle le catalyseur de réaction de gaz à l'eau inverse comprend Fe, Co, Cu, Cr, Ni, Ir, Mn ou des mélanges de ceux-ci.

5. . Procédé selon l'une quelconque des revendications précédentes, dans laquelle le catalyseur Fischer-Tropsch comprend du Fe et/ou du Co, de préférence du Co.

6. . Procédé selon l'une quelconque des revendications précédentes, dans lequel le catalyseur à base de zéolithe à l'étape (iii) comprend une zéolithe de type MFI, une zéolithe de type CHA, une zéolithe de type BEA, une zéolithe de type MOR, une zéolithe de type FAU, une zéolithe de type MEL, une zéolithe de type FER, une zéolithe de type MTT, une zéolithe de type TON, une zéolithe de type ERI, une zéolithe de type MTW, une zéolithe de type MWW ou un mélange ceux-ci.

7. . Procédé selon l'une quelconque des revendications précédentes, dans lequel un autre catalyseur à base de zéolithe modifiée par un métal est présent à l'étape (i).

8. . Procédé selon l'une quelconque des revendications précédentes, dans laquelle l'étape (ii) produit en outre des hydrocarbures saturés en C7+, de préférence des hydrocarbures saturés en C8+.

9. . Procédé selon l'une quelconque des revendications précédentes, dans laquelle, à l'étape (i), une charge comprenant le CO₂ est introduite dans le catalyseur de déplacement de gaz à l'eau inverse, la charge étant exempte de CO de manière substantielle.

10. . Procédé selon l'une quelconque des revendications précédentes,
dans lequel, à l'étape (i), le CO₂ est réagi avec H₂ à une température de 250 à 1000°C à l'aide d'un catalyseur de réaction de gaz á l'eau inverse comprenant Ni/Al₂O₃ pour produire un mélange comprenant du CO et H₂O,
dans lequel H₂O est au moins partiellement séparé du CO dans une étape de refroidissement ultérieure avant la conversion du CO à l'étape (ii),
dans lequel, à l'étape (ii), le CO de l'étape (i) est réagi avec H₂ à l'aide d'un catalyseur Fischer-Tropsch comprenant du Co pour produire des hydrocarbures saturés C8+ et des hydrocarbures insaturés comprenant au moins l'un des éthylène, propylène et/ou butylène, et
dans lequel, à l'étape (iii), le catalyseur à base de zéolithe comprend une zéolithe de type MFI ou une zéolithe de type BEA.
